# EUROPÄISCHE PATENTSCHRIFT

(11) **EP 4 135 810 B1**
(45) Veröffentlichungstag und Bekanntmachung des Hinweises auf die Patenterteilung: **11.10.2023**
(21) Anmeldenummer: 21734046.2
(22) Anmeldetag: 17.06.2021
(51) Int. Cl.: A61M 16/04

(54) **TRACHEALKANÜLE**
TRACHEAL CANNULA
CANULE TRACHÉALE

(30) Priorität: 17.06.2020 DE 102020115938
(43) Veröffentlichungstag der Anmeldung: 22.02.2023
(73) Patentinhaber: Andreas Fahl Medizintechnik-Vertrieb GmbH, 51149 Köln (DE)
(72) Erfinder: FAHL, Andreas, 51149 Köln (DE)
(74) Vertreter: Geskes, Christoph
(86) Internationale Anmeldenummer: PCT/EP2021/066361
(87) Internationale Veröffentlichungsnummer: WO 2021/255152

(56) Entgegenhaltungen:
- EP-B1- 1 003 584
- GB-A- 2 329 340
- US-A- 2 039 142
- US-A1- 2002 029 782
- US-A1- 2004 226 564
- US-A1- 2019 184 120
- US-B2- 10 894 137

## Beschreibung

Die Erfindung betrifft eine Trachealkanüle für Laryngektomierte und/oder Tracheotomierte umfassend ein Kanülenrohrteil, ein Konnektorteil und zumindest zwei Trageflügel zur Befestigung eines Tragebandes.

Trachealkanülen sind aus dem Stand der Technik allgemein bekannt. So beschreibt DE 10 2009 013 424 A1 eine Trachealkanüle mit einem horizontalem Lochsieb. Weitere Trachealkanülen sind aus US 2004/226564 A1, GB 2 329 340 A, EP 1 003 584 B1 und US 2002/029782 A1 bekannt.

Die aus dem Stand der Technik bekannten Trachealkanülen haben den Nachteil, dass sie insbesondere bei bestimmen Tracheostomaformen nur unzureichend abdichten.

Aufgabe der Erfindung ist es daher eine verbesserte Trachealkanüle zur Verfügung zu stellen, die insbesondere die aus dem Stand der Technik bekannten Nachteile überkommt. Insbesondere ist die Aufgabe der Erfindung eine Trachealkanüle mit einem verbesserten Tragekomfort zur Verfügung zu stellen. Insbesondere ist die Aufgabe der Erfindung eine Trachealkanüle mit einer verbesserten Abdichtung zum Tracheostoma umgebenden Gewebe zur Verfügung zu stellen.

Die Aufgabe wird erfindungsgemäß gelöst mittels einer Trachealkanüle für Laryngektomierte und/oder Tracheotomierte umfassend ein Kanülenrohrteil, ein Konnektorteil und zumindest zwei Trageflügel zur Befestigung eines Tragebandes, wobei Konnektorteil und Kanülenrohrteil einstückig ausgebildet sind, wobei die Trageflügel an dem Konnektorteil angeordnet sind, wobei das Konnektorteil einen ersten Kegelteil und einen zweiten Kegelteil umfasst, wobei der erste Kegelteil an dem Kanülenrohrteil angeordnet ist und sich von dem Kanülenrohrteil weg aufweitet, und wobei der zweite Kegelteil an dem ersten Kegelteil angeordnet ist und sich zum ersten Kegelteil hin aufweitet.

Es wird eine Trachealkanüle für Laryngektomierte und/oder Tracheotomierte vorgeschlagen. Diese umfasst ein Kanülenrohrteil, ein Konnektorteil und zumindest zwei Trageflügel zur Befestigung eines Tragebandes, wobei Konnektorteil und Kanülenrohrteil einstückig ausgebildet sind. Die Trageflügel sind an dem Konnektorteil angeordnet, wobei das Konnektorteil einen ersten Kegelteil und einen zweiten Kegelteil umfasst. Der erste Kegelteil ist an dem Kanülenrohrteil angeordnet und weitet sich von dem Kanülenrohrteil weg auf. Der zweite Kegelteil ist an dem ersten Kegelteil angeordnet und weitet sich zum ersten Kegelteil hin auf.

Bevorzugt verjüngt sich das erste Kegelteil zum Kanülenrohrteil hin. Weiter bevorzugt ist ein kleinster Durchmesser des ersten Kegelteils etwa gleich einem Durchmesser der Trachealkanüle, bevorzugt ein Durchmesser der Trachealkanüle an deren dem ersten Kegelteil zugewandten Ende.

Wird im Rahmen der Erfindung der Begriff "etwa" im Zusammenhang mit Werten oder Wertebereichen verwendet, so ist darunter ein Toleranzbereich zu verstehen, den der Fachmann auf diesem Gebiet für üblich erachtet, insbesondere ist ein Toleranzbereich von ±20 %, bevorzugt ±10 %, weiter bevorzugt ±5 % vorgesehen.

Soweit verschiedene Wertebereiche, beispielsweise bevorzugte und weiter bevorzugte Wertebereiche, in der vorliegenden Erfindung angegeben sind, sind die Untergrenzen und die Obergrenzen der verschiedenen Wertebereiche miteinander kombinierbar.

Bevorzugt verjüngt sich das zweite Kegelteil vom ersten Kegelteil weg. Weiter bevorzugt ist ein größter Durchmesser des zweiten Kegelteils etwa gleich einem größten Durchmesser des ersten Kegelteils, der weiter bevorzugt unmittelbar an dem zweiten Kegelteil angrenzt.

Im Sinne der Erfindung ist ein Durchmesser ein Innendurchmesser und/oder ein Außendurchmesser, besonders bevorzugt eine Außendurchmesser. Bevorzugt ist ein kleinster Außendurchmesser des ersten Kegelteils etwa gleich einem Außendurchmesser der Trachealkanüle, bevorzugt ein Außendurchmesser der Trachealkanüle an deren dem ersten Kegelteil zugewandten Ende. Weiter bevorzugt ist ein größter Außendurchmesser des zweiten Kegelteils etwa gleich einem größten Außendurchmesser des ersten Kegelteils, der weiter bevorzugt unmittelbar an dem zweiten Kegelteil angrenzt.

Die Trachealkanüle umfasst ein Kanülenrohrteil. In einer Ausgestaltung ist das Kanülenrohrteil gefenstert oder gesiebt ausgeführt. In einer weiteren Ausgestaltung umfasst das Kanülenrohrteil eine horizontale Siebung. In einer weiteren Ausgestaltung ist das Kanülenrohrteil ungefenstert und ungesiebt ausgeführt.

Bevorzugt ist eine Länge des Kanülenrohrteils etwa 36 mm bis etwa 55 mm. Bevorzugt ist die Länge des Kanülenrohrteils ein Abstand vom Konnektorteil bis zum dem Konnektorteil gegenüberliegenden proximalen Ende.

Werden im Rahmen der Beschreibung der Erfindung Richtungsangaben verwendet, so sind diese in Bezug auf den üblichen Gebrauch der Trachealkanüle zu verstehen. Richtungsbezeichnungen werden wie in der Anatomie üblich verwendet. Insbesondere können medizinische Richtungsangaben auch dann verwendet werden, wenn die Trachealkanüle nicht von einem Benutzer getragen wird. Hierbei wird die übliche Verwendung der Trachealkanüle angenommen, um die Richtung zu bestimmen. Unter dem Begriff "distal" wird im Sinne der vorliegenden Erfindung in Bezug auf ein Merkmal der erfindungsgemäßen Vorrichtung eine Anordnung bzw. Verwendung desselben fern oder auch abgewandt oder gegenüberliegend der Trachealkanüle, allgemeiner einer Hautoberfläche einer Person, die insbesondere die Trachealkanüle trägt, zuordenbar verstanden. Unter distal ist vorzugsweise eine Richtung vom Körper des Benutzers weg zu verstehen, bei vorgesehener Verwendung der Trachealkanüle. Unter dem Begriff "proximal" wird im Sinne der vorliegenden Erfindung in Bezug auf ein Merkmal der erfindungsgemäßen Vorrichtung eine Anordnung oder Verwendung desselben nah oder auch zugewandt oder benachbart einer Trachealkanüle, allgemeiner zum Körperzentrum einer Person, die die Trachealkanüle trägt, zuordenbar verstanden. Unter proximal ist vorzugsweise eine Richtung zum Körperzentrum des Benutzers hin zu verstehen, bei vorgesehener Verwendung der Trachealkanüle. Beispielsweise ragt der Kanülenrohrteil vom Konnektorteil ausgehend nach distal und kaudal - zum Gesäß gerichtet. Der Konnektorteil ist beispielsweise distal von dem Kanülenrohrteil angeordnet.

Bevorzugt ist ein Außendurchmesser des Kanülenrohrteils etwa 8 bis etwa 12 mm, bevorzugt etwa 8 mm, weiter bevorzugt etwa 9 mm, weiter bevorzugt etwa 10 mm, weiter bevorzugt etwa 12 mm.

In einer Ausgestaltung ist das Kanülenrohrteil aus einem Material ausgewählt aus einer Gruppe zumindest umfassend Silikon und/oder TPE. In einer weiteren Ausgestaltung ist vorgesehen, dass zumindest das Kanülenrohrteil (20) eine Härte von etwa 60 Shore-A bis etwa 80 Shore-A nach DIN ISO 7619-1 (2012-02) umfasst. Bevorzugt ist vorgesehen, dass das Kanülenrohrteil eine Härte von etwa 70 Shore-A umfasst.

Die Trachealkanüle umfasst des Weiteren einen Konnektorteil. In einer Ausgestaltung ist das Konnektorteil aus einem Material ausgewählt aus einer Gruppe zumindest umfassend Silikon und/oder TPE. In einer Ausgestaltung umfasst das Konnektorteil dasselbe Material wie das Kanülenrohrteil. In einer weiteren Ausgestaltung weist das Konnektorteil ein anderes Material auf als das Kanülenrohrteil. Erfindungsgemäß sind Konnektorteil und Kanülenrohrteil einstückig ausgebildet, bevorzugt in einem Produktionsschritt gespritzt.

In einer weiteren Ausgestaltung ist vorgesehen, dass zumindest das Konnektorteil eine Härte von etwa 60 Shore-A bis etwa 80 Shore-A nach DIN ISO 7619-1 (2012-02) umfasst. Bevorzugt ist vorgesehen, dass das Konnektorteil eine Härte von etwa 70 Shore-A umfasst. In einer Ausgestaltung ist vorgesehen, dass das Konnektorteil eine andere Härte als das Kanülenrohrteil umfasst, insbesondere härter ist als das Kanülenrohrteil. In einer weiteren Ausgestaltung umfasst das Kanülenrohrteil und das Konnektorteil etwa die gleiche Härte.

Das Konnektorteil umfasst den ersten Kegelteil. In einer Ausgestaltung umfasst der erste Kegelteil einen ersten halben Öffnungswinkel. Der erste halbe Öffnungswinkel beträgt bevorzugt etwa 20° bis etwa 45°, weiter bevorzugt etwa 38°.

Im Sinne der Erfindung ist der halbe Öffnungswinkel ein Winkel zwischen den Mantellinien einer Außenumhüllenden, das heißt den Verbindungsstrecken der Punkte eines Basiskreises des ersten oder zweiten Kegelteils mit einer Spitze des jeweiligen Kegelteils, und einer Hochachse des ersten beziehungsweise zweiten Kegelteils.

Bevorzugt umfasst das erste Kegelteil eine durchgängige Ausnehmung insbesondere von proximal nach distal, weiter bevorzugt von der Seite, die dem ersten Kanülenrohrteil zugewandt ist zu der gegenüberliegenden Seite, die dem zweiten Kegelteil zugewandt ist. Weiter bevorzugt ist das erste Kegelteil kommunizierend mit dem Kanülenrohrteil verbunden. Die Ausnehmung wird in einer Ausgestaltung begrenzt durch eine bevorzugt im Wesentlichen kegelförmige Innenwandung des ersten Kegelteils.

Der Begriff "im Wesentlichen" gibt einen Toleranzbereich an, der für den Fachmann unter wirtschaftlichen und technischen Gesichtspunkten zu vertreten ist, so dass das entsprechende Merkmal noch als solches zu erkennen oder verwirklicht ist.

In einer Ausgestaltung ist vorgesehen, dass das erste Kegelteil eine erste Materialdicke aufweist. Bevorzugt ist die erste Materialdicke zumindest Abschnittsweise etwa gleich einer Materialdicke des Kanülenrohrteils. Weiter bevorzugt ist die Materialdicke des Kanülenrohrteils etwa 1 mm bis etwa 1,5 mm. Weiter bevorzugt ist die Materialdicke des ersten Kegelteils zumindest Abschnittsweise etwa 1 mm bis etwa 1,5 mm.

Das Konnektorteil umfasst den zweiten Kegelteil. In einer Ausgestaltung umfasst der zweite Kegelteil einen zweiten halben Öffnungswinkel. Der zweite halbe Öffnungswinkel beträgt bevorzugt etwa 10° bis etwa 45°, weiter bevorzugt etwa 16°.

Bevorzugt umfasst das zweite Kegelteil eine durchgängige Ausnehmung bevorzugt von proximal nach distal, weiter bevorzugt von der Seite, die dem ersten Kegelteil zugewandt ist zu der gegenüberliegenden Seite. Weiter bevorzugt ist das zweite Kegelteil kommunizierend mit dem ersten Kegelteil verbunden. Die Ausnehmung wird in einer Ausgestaltung begrenzt durch eine bevorzugt im Wesentlichen kegelförmige Innenwandung des zweiten Kegelteils. In einer weiteren Ausgestaltung weist die Innenwandung des zweiten Kegelteils ein Konnektorprofil für ein Tracheostomahilfsmittel auf. Bevorzugt umfasst das Konnektorprofil einen Konnektoröffnungsdurchmesser von etwa 22 mm. Bevorzugt bildet das zweite Kegelteil eine Standardaufnahme für Tracheostomahilfsmittel. Weiter bevorzugt ist das Konnektorprofil zur Aufnahme eines Tracheostomahilfsmittels mit einem Durchmesser von etwa 22 mm ausgestaltet. Tracheostomahilfsmittel sind beispielsweise Wärme-Feuchtigkeitsaustauscher, Sprechventile und/oder Beatmungsmittel.

Beispielhafte Aufzählungen sind im Sinne der Erfindung als nicht abschließend anzusehen, sondern können im Rahmen des allgemeinen Fachwissens ergänzt werden.

In einer Ausgestaltung ist vorgesehen, dass das zweite Kegelteil eine zweite Materialdicke aufweist. Bevorzugt ist die zweite Materialdicke zumindest Abschnittsweise gleich der Materialdicke des ersten Kegelteils. Weiter bevorzugt ist die Materialdicke des zweiten Kegelteils zumindest Abschnittsweise etwa 1 mm bis etwa 2,5 mm, weiter bevorzugt etwa 1,5 mm bis etwa 2,5 mm.

Im Sinne der Erfindung ist ein Kegel ein geometrischer Körper, bei dem alle Punkte einer Grundfläche gradlinig mit einer Spitze beziehungsweise einem Punkt einer Ebene, in der nicht die Grundfläche angeordnet ist, verbunden sind. Die Grundfläche kann kreisförmig, rechteckig, oval oder jede beliebige andere Form, insbesondere mit abgerundeten Ecken, aufweisen. Weiterhin weist im Sinne der Erfindung die Ausnehmung des ersten und/oder des zweiten Kegelteils bevorzugt eine kreiskegelförmige Ausgestaltung auf. Unter einem Kegel kann im Sinne der Erfindung auch ein Kegelstumpf verstanden werden. Insbesondere ist vorgesehen, dass die Ausnehmung das erste und/oder zweite Kegelteil derart überlagert, dass das erste und/oder zweite Kegelteil als Kegelstumpf ausgebildete ist. In einer Ausgestaltung ist eine Längsachse der Ausnehmung vorzugsweise mit der Hochachse des ersten und/oder zweiten Kegelteils, die ein Lot von einer theoretischen, nicht ausgebildeten Spitze auf die Grundfläche der Kegelform des ersten und/oder zweiten Kegelteils ist, kongruent.

In einer Ausgestaltung ist vorgesehen, dass der erste Kegelteil und der zweite Kegelteil über einen Übergangsbereich aneinander angrenzen. Bevorzugt ist im Übergangsbereich ein Radius, bevorzugt auf einer Außenoberfläche des Konnektorteils, zwischen dem ersten Kegelteil und dem zweiten Kegelteil ausgebildet. Weiter bevorzugt weist der Übergangsbereich eine Übergangsstelle auf. Die Übergangsstelle ist als Wendepunkt bevorzugt der Außenoberfläche des Übergangsbereiches in Ansicht in einem Längsschnitt durch die Trachealkanüle ausgebildet. In einer Ausgestaltung, in der der erste Kegelteil und der zweite Kegelteil unmittelbar aneinandergrenzen und bevorzugt der Konnektor keinen Übergangsbereich aufweist, ist die Übergangsstelle, die im Wesentlichen unstete Stelle auf der Außenoberfläche, bei der erstes Kegelteil und zweites Kegelteil aneinandergrenzen. Bevorzugt umfasst der Übergangsbereich bevorzugt auf der Außenoberfläche einen Radius von etwa 3 mm bis etwa 10 mm, weiter bevorzugt etwa 5 mm auf. In einer Ausgestaltung weist der Konnektorteil an der Übergangsstelle einen maximalen Durchmesser, insbesondere einen maximalen Außendurchmesser auf.

Bevorzugt ist die Übergangsstelle auf einer Frontalebene angeordnet. Die Frontalebene ist bevorzugt die Ebene, die voraussichtlich eine Hautoberfläche eines Benutzers der Trachealkanüle zumindest tangiert, wenn die Trachealkanüle im Tracheostoma des Benutzers angeordnet ist.

Die Trachealkanüle umfasst Trageflügel. Die Trageflügel ersetzen vorteilhaft ein den Konnektorteil vollständig umlaufendes Kanülenschild und sind somit wesentlich komfortabler und optisch ansprechender als bei aus dem Stand der Technik bekannten Trachealkanülen. In einer Ausgestaltung ist vorgesehen, dass die Trageflügel im Wesentlichen im Übergangsbereich angeordnet sind. Bevorzugt sind die Trageflügel lateral am Konnektorteil angeordnet. Weiter bevorzugt umfasst das Konnektorteil bevorzugt im Übergangsbereich eine Anschlussstelle, an der die Trageflügel angeordnet sind. Weiter bevorzugt sind die Trageflügel mit dem Konnektorteil materialverbunden. In einer weiteren Ausgestaltung ist vorgesehen, dass das Konnektorteil zumindest im Bereich der Anschlussstelle eine größere Materialdicke aufweist als in anderen Bereichen des Konnektorteils. In einer weiteren Ausgestaltung ist die Materialdicke des zweiten Kegelteils dicker als diejenige des ersten Kegelteils. In einer weiteren Ausgestaltung ist vorgesehen, dass die Anschlussstelle respektive die Trageflügel nur an dem zweiten Kegelteil angeordnet sind. Weiter bevorzugt ist die Anschlussstelle distal der Übergangsstelle angeordnet.

Die Trageflügel sind zur Aufnahme eines Tragebandes ausgestaltet, mittels dem die Trachealkanüle am Hals haltbar ist. Weiter bevorzugt ist in einer Ausgestaltung vorgesehen, dass die Trageflügel jeweils zumindest eine Bandausnehmung aufweisen. In einer weiteren Ausgestaltung ist vorgesehen, dass die Bandausnehmungen bevorzugt vollständig durchgehend durch die Trageflügel ausgestaltet sind. Bevorzugt ist eine Weite und/oder Breite der Bandausnehmung an das voraussichtlich zu verwendende Trageband angepasst, bevorzugt derart, dass das Trageband im Wesentlichen kein Spiel hat, sich zu bewegen. Hierdurch werden Bewegungen des Tragbandes vorteilhaft verhindert, insbesondere um ein Scheuern auf der Haut zu verhindern.

Die Tragflügel erstrecken sich in einer Ausgestaltung von der Anschlussstelle nach lateral. Weiter bevorzugt erstrecken sich die Trageflügel von der Anschlussstelle nach lateral und proximal. In einer Ausgestaltung ist vorgesehen, dass die Trageflügel gekrümmt sind. Bevorzugt sind diese geschwungen ausgestaltet beziehungsweise weisen weiter bevorzugt einen Radius auf. Bevorzugt sind die Trageflügel in Richtung des Kanülenrohrteils gekrümmt.

In einer weiteren Ausgestaltung ist vorgesehen, dass ein Radius einer Krümmung der Trageflügel im Wesentlichen in einer Transversalebene anordenbar ist. Die Transversalebene entspricht der Horizontalebene eines stehenden Benutzers.

In einer Ausgestaltung ist vorgesehen, dass die Trageflügel ausgehend von einer Anschlussstelle am Konnektorteil sich nach distal und nach lateral erstrecken. Bevorzugt sind die Trageflügel im Bereich der Anschlussstelle eher nach lateral gerichtet und im Bereich eines lateral gelegenen Endes der Trageflügel eher nach distal gerichtet. Weiter bevorzugt wird über die Länge des Trageflügels ausgehend von der Anschlussstelle ein Vektor der Erstreckung nach distal größer. Weiter bevorzugt wird über die Länge des Trageflügels ausgehend von der Anschlussstelle ein Vektor der Erstreckung nach lateral kleiner.

In einer Ausgestaltung ist vorgesehen, dass ein von der Anschlussstelle abgewandtes Ende der Trageflügel einen Abstand zu einer Frontalebene, auf der eine im Übergangsbereich angeordnete Übergangsstelle angeordnet ist, umfasst. Durch diese Ausgestaltung wird vorteilhaft verhindert, dass die Trageflügel die Hautoberfläche des Benutzers berühren. Weiterhin bevorzugt wird in einer Ausgestaltung vorgeschlagen, dass der Abstand etwa einer Breite mindestens einer in den Tragflügeln angeordneten Bandausnehmung entspricht. Bevorzugt ist der Abstand gleich einer Dicke eines vorgesehenen Tragebandes. Auf diese Weise wird sichergestellt, dass das Trageband mit seiner dem Kanülenrohrteil zugewandten Seite, weiter bevorzugt mit seiner proximalen Seite zumindest teilweise kongruent mit der Frontalebene ist. Weiter bevorzugt ist durch diese Ausgestaltung sichergestellt, dass das Trageband flach auf der Hautoberfläche den Benutzers aufliegt und keine Druckstellen auslöst.

Bevorzugt ist vorgesehen, dass ein Abstand der Anschlussstelle zur Frontalebene der Übergangsstelle beziehungsweise des Wendebereiches der Übergangsstelle etwa gleich einer Breite der Bandausnehmung, weiter bevorzugt eines vorgesehenen Tragebandes ist, das mit der Trachealkanüle verwendet werden kann. In einer Ausgestaltung ist vorgesehen, dass der Abstand etwa einer Breite mindestens einer in den Tragflügeln angeordneten Bandausnehmung entspricht. Weiter bevorzugt ist ein Abstand der Anschlussstelle zur Frontalebene der Übergangsstelle beziehungsweise des Wendebereiches der Übergangsstelle etwa 1 mm bis etwa 5 mm, bevorzugt etwa 2 mm.

Bevorzugt ist die Bandausnehmung im Bereich einer Wendestelle des jeweiligen Trageflügels angeordnet.

In einer weiteren Ausgestaltung ist vorgesehen, dass die Trachealkanüle eine Kennzeichnung umfasst. In einer weiteren Ausgestaltung ist vorgesehen, dass die Kennzeichnung eine Größe und/oder eine Länge der Trachealkanüle kennzeichnet. Bevorzugt ist die Kennzeichnung auf einer Außenseite eines Konnektorteils angeordnet. Weiter bevorzugt ist eine Kennzeichnung auf dem zweiten Kegelteil angeordnet. Die Anordnung der Kennzeichnung auf dem zweiten Kegelteil hat den Vorteil, dass bei üblicher Lagerung der Trachealkanülen diese nicht angefasst werden braucht, um die Größe der Trachealkanüle zu bestimmen. Insbesondere durch die Kegelform des zweiten Kegelteils ist die Kennzeichnung auch in der Verpackung zu erkennen, da diese nicht durch umgebenes Packungsmaterial verdeckt wird.

Eine beispielhafte Ausgestaltung der Trachealkanüle umfasst einen Kanülenrohrteil und einen Konnektorteil. Der Konnektorteil ist mit dem Kanülenrohrteil materialverbunden. Der Konnektorteil umfasst einen ersten Kegelteil und einen zweiten Kegelteil. Am Konnektorteil sind des Weiteren zwei Trageflügel angeordnet, die sich von einer Anschlussstelle im Wesentlichen nach lateral erstrecken. Die Trageflügel weisen jeweils eine Bandausnehmung auf, durch die ein Trageband führbar ist. Eine Breite der Bandausnehmungen ist jeweils derart ausgeführt, dass diese etwa einer Dicke des Tragebandes entspricht. Auch die Weite des Tragebandes ist derart ausgelegt, dass diese etwa einer Breite des Tragebandes entspricht.

Der zweite Kegelteil weist eine durchgängige zweite Ausnehmung auf, die mit einer ersten Ausnehmung des ersten Kegelteils kommuniziert, die wiederum mit dem Kanülenrohrteil kommuniziert. Weiterhin weist der zweite Kegelteil eine Innenwandung auf, die ein Konnektorprofil umfasst. Mittels des Konnektorprofils kann ein hier nicht dargestelltes Tracheostomahilfsmittel, beispielsweise ein Feuchte-Wärme-Austauscher, an der Trachealkanüle befestigt werden. Auf dem zweiten Kegelteil ist, durch die Kegelform von vorne gut sichtbar, eine Kennzeichnung aufgebracht, die bevorzugt zumindest eine Größe der Trachealkanüle zeigt.

Das Konnektorteil, respektive der erste Kegelteil und der zweite Kegelteil sowie der Kanülenrohrteil sind in der beispielhaften Ausgestaltung materialverbunden ausgestaltet. Insbesondere ist die Trachealkanüle aus einem Silikon mit einer Härte von etwa 70 Shore-A gefertigt. Zwischen dem ersten Kegelteil und dem zweiten Kegelteil ist ein Übergangsbereich ausgebildet, der einen beispielhaft Radius aufweist. Ein Wendepunkt des Übergangsbereiches markiert eine Übergangsstelle zwischen dem ersten Kegelteil und dem zweiten Kegelteil, die gleichzeitig bevorzugt eine maximale Eindringtiefe der Trachealkanüle in das Tracheostoma eines Benutzers darstellt. Die Frontalebene, auf der die Übergangsstelle angeordnet ist, ist bevorzugt zumindest teilweise die Ebene, der Hautoberfläche des Benutzers, wenn die Trachealkanüle in das Tracheostoma eingesetzt ist. Die Trageflügel sind im Wesentlichen distal der Frontalebene angeordnet, so dass Hautirritationen und Druckstellen durch die Trageflügel vermieden werden können.

Der erste Kegelteil weist beispielhaft einen ersten halben Öffnungswinkel zwischen der Hochachse und einer Mantellinie einer Außenumhüllenden des ersten Kegelteils von etwa 38° auf. Der zweite Kegelteil, dessen Grundfläche der Grundfläche des ersten Kegelteils zugeordnet ist, weist zwischen der Hochachse und einer Mantellinie einer Außenumhüllenden des zweiten Kegelteils einen beispielhaften zweiten halben Öffnungswinkel von etwa 16° auf. In der beispielhaften Ausgestaltung sind die Hochachse des ersten Kegelteiles und die Hochachse des zweiten Kegelteils und die Längsachsen der ersten Ausnehmung und der zweiten Ausnehmung identisch. Diese Anordnung der Kegelteile ist optisch ansprechend und besonders komfortabel beim Tragen der Trachealkanüle.

Der zweite Kegelteil umfasst beispielhaft eine zweite Materialdicke, die größer ist als eine erste Materialdicke des ersten Kegelteils. Der zweite Kegelteil umfasst das Konnektorprofil und ist vorteilhaft verstärkt, um die Trageflügel zu halten, ohne dass bei einer Benutzung das Konnektorteil wesentlich verformt wird. Hingegen ist die erste Materialdicke derart ausgelegt, dass leichte Verformungen zur Anpassung an das das Tracheostoma umgebene Gewebe möglich ist.

Weitere vorteilhafte Ausgestaltungen gehen aus den nachfolgenden Zeichnungen hervor. Die dort dargestellten Weiterbildungen sind jedoch nicht beschränkend auszulegen, vielmehr können die dort beschriebenen Merkmale untereinander und mit den oben beschriebenen Merkmalen zu weiteren Ausgestaltungen kombiniert werden. Des Weiteren sei darauf verwiesen, dass die in der Figurenbeschreibung angegebenen Bezugszeichen den Schutzbereich der vorliegenden Erfindung nicht beschränken, sondern lediglich auf die in den Zeichnungen gezeigten Ausführungsbeispiele verweisen. Gleiche Teile oder Teile mit gleicher Funktion weisen im Folgenden die gleichen Bezugszeichen auf. Es zeigen:
Fig. 1 eine Trachealkanüle in einer ersten Ansicht; und
Fig. 2 die Trachealkanüle in einer Seitenansicht.

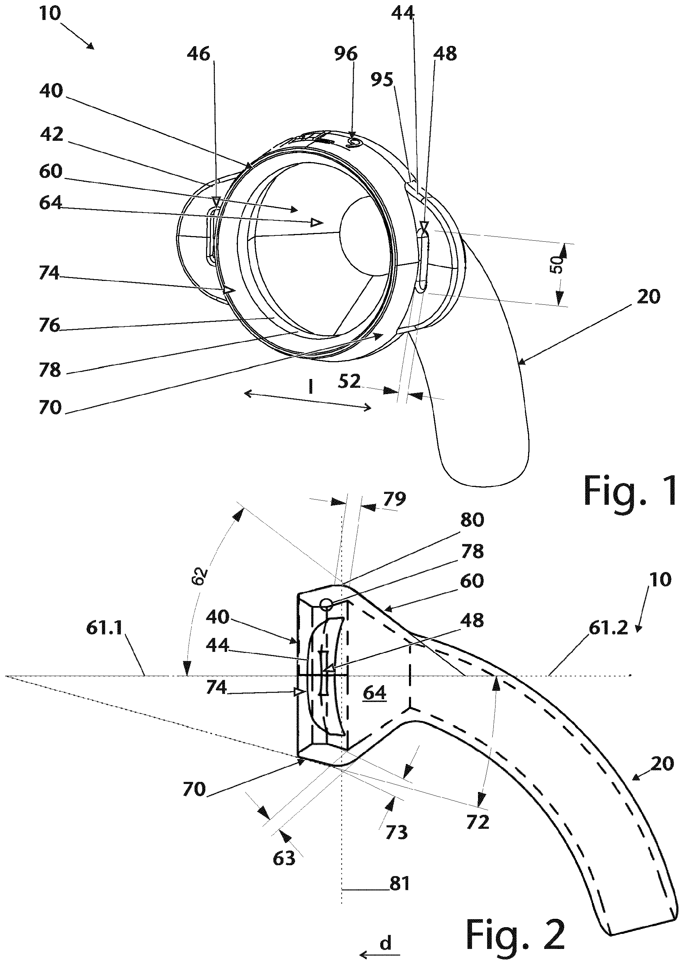

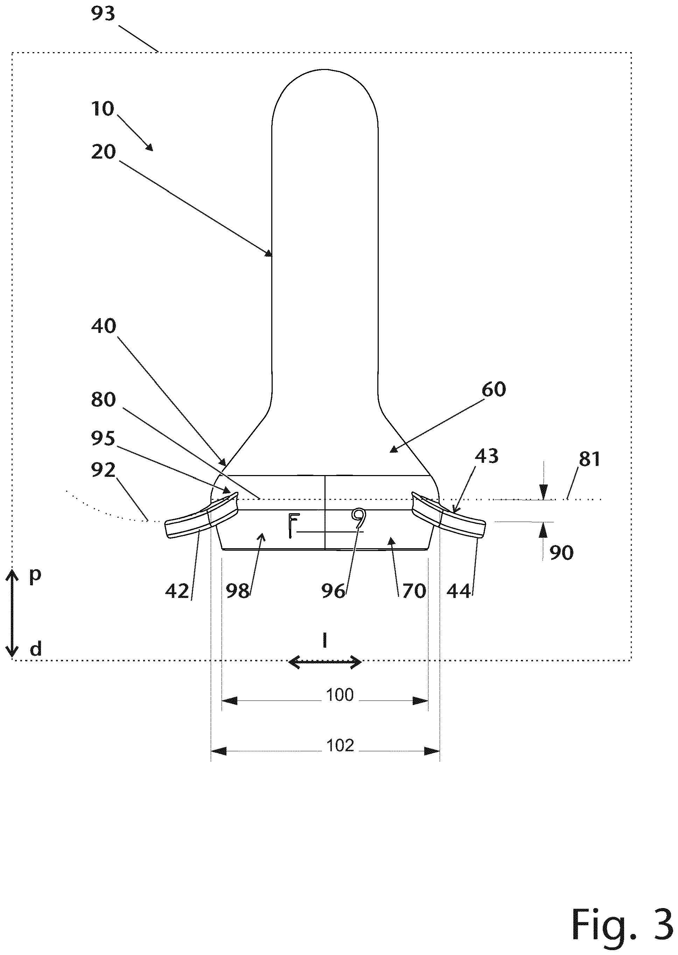

Fig. 1 zeigt eine Trachealkanüle 10 umfassend einen Kanülenrohrteil 20 und einen Konnektorteil 40. Der Konnektorteil 40 ist mit dem Kanülenrohrteil 20 materialverbunden. Der Kanülenrohrteil 40 umfasst einen ersten Kegelteil 60 und einen zweiten Kegelteil 70. Am Konnektorteil 40 sind des Weiteren zwei Trageflügel 42, 44 angeordnet, die sich von einer Anschlussstelle 95 im Wesentlichen nach lateral l erstrecken. Die Trageflügel 42, 44 weisen jeweils eine Bandausnehmung 46, 48 auf, durch die ein hier nicht gezeigtes Trageband führbar ist. Eine Breite 52 der Bandausnehmungen 46, 48 ist jeweils derart ausgeführt, dass diese etwa einer Dicke des Tragebandes entspricht. Auch die Weite 50 des Tragebandes ist derart ausgelegt, dass diese etwa einer Breite des Tragebandes entspricht.

Der zweite Kegelteil 70 weist eine durchgängige zweite Ausnehmung 74 auf, die mit einer ersten Ausnehmung 64 des ersten Kegelteils kommuniziert, die wiederum mit dem Kanülenrohrteil 20 kommuniziert. Weiterhin weist der zweite Kegelteil 70 eine Innenwandung 76 auf, die ein Konnektorprofil 78 umfasst. Mittels des Konnektorprofils 78 kann ein hier nicht dargestelltes Tracheostomahilfsmittel, beispielsweise ein Feuchte-Wärme-Austauscher, an der Trachealkanüle 10 befestigt werden. Auf dem zweiten Kegelteil 70 ist, durch die Kegelform von vorne gut sichtbar, eine Kennzeichnung 96 aufgebracht, die bevorzugt zumindest eine Größe der Trachealkanüle 10 zeigt.

Fig. 2 zeigt die Trachealkanüle 10 in einer Seitenansicht, mit gestrichelt angedeuteten verdeckten Kanten. Das Konnektorteil 40, respektive der erste Kegelteil 60 und der zweite Kegelteil 70 sowie der Kanülenrohrteil 20 sind materialverbunden ausgestaltet. Insbesondere ist die Trachealkanüle aus einem Silikon mit einer Härte von etwa 70 Shore-A gefertigt. Zwischen dem ersten Kegelteil 60 und dem zweiten Kegelteil 70 ist ein Übergangsbereich 79 ausgebildet, der einen hier nicht weiter bezeichneten Radius aufweist. Ein Wendepunkt des Übergangsbereiches 79 markiert eine Übergangsstelle 80, die gleichzeitig bevorzugt eine maximale Eindringtiefe der Trachealkanüle 10 in das Tracheostoma eines Benutzers darstellt. Die Frontalebene 81, auf der die Übergangsstelle 89 angeordnet ist, ist bevorzugt zumindest teilweise die Ebene, der Hautoberfläche des Benutzers, wenn die Trachealkanüle 10 in das Tracheostoma eingesetzt ist. Die Trageflügel 46, 48 sind im Wesentlichen distal d der Frontalebene angeordnet, so dass Hautirritationen und Druckstellen durch die Trageflügel 42, 44 vermieden werden können.

Der erste Kegelteil 60 weist einen ersten halben Öffnungswinkel 62 zwischen einer Hochachse 61.1 des ersten Kegelteils 60 und einer nicht bezeichneten Mantellinie einer Außenumhüllenden des ersten Kegelteils 60 von etwa 38° auf. Der zweite Kegelteil 70, dessen hier nicht gezeigte Grundfläche der nicht gezeigten Grundfläche des ersten Kegelteils 60 zugeordnet ist, weist zwischen einer Hochachse 61.2 des zweiten Kegelteils 70 und einer nicht bezeichneten Mantellinie einer Außenumhüllenden des zweiten Kegelteils 70 einen zweiten halben Öffnungswinkel 72 von etwa 16° auf. In der in Fig. 2 gezeigten Ausgestaltung sind die Hochachse 61.1 des ersten Kegelteiles 60 und die Hochachse 62.2 des zweiten Kegelteils 70 und die nicht weiter bezeichnete Längsachsen der ersten Ausnehmung 64 und der zweiten Ausnehmung 74 identisch. Diese Anordnung der Kegelteile 60 und 70 ist optisch ansprechend und insbesondere komfortabel beim Tragen der Trachealkanüle.

Der zweite Kegelteil 70 umfasst eine zweite Materialdicke 73, die größer ist als eine erste Materialdicke 63 des ersten Kegelteils 60. Der zweite Kegelteil 70 umfasst das in Fig. 1 bezeichnete Konnektorprofil 78 und ist vorteilhaft verstärkt, um die Trageflügel 42, 44 zu halten, ohne dass bei einer Benutzung das Konnektorteil 40 wesentlich verformt wird. Hingegen ist die erste Materialdicke 63 derart ausgelegt, dass leichte Verformungen zur Anpassung an das das Tracheostoma umgebene Gewebe möglich ist.

Die vorgeschlagene Trachealkanüle 10 kann komfortabel getragen werden und vermeidet durch ihre Geometrie Druckstellen und Hautirritationen.

## Patentansprüche

1. Trachealkanüle (10) für Laryngektomierte und/oder Tracheotomierte umfassend ein Kanülenrohrteil (20), ein Konnektorteil (40) und zumindest zwei Trageflügel (42, 44) zur Befestigung eines Tragebandes, wobei die Trageflügel (42, 44) an dem Konnektorteil (40) angeordnet sind, wobei das Konnektorteil (40) einen ersten Kegelteil (60) und einen zweiten Kegelteil (70) umfasst, wobei der erste Kegelteil (60) an dem Kanülenrohrteil (20) angeordnet ist und sich von dem Kanülenrohrteil (20) weg aufweitet, und wobei der zweite Kegelteil (70) an dem ersten Kegelteil (60) angeordnet ist und sich zum ersten Kegelteil (60) hin aufweitet, **gekennzeichnet dadurch, dass** Konnektorteil (40) und Kanülenrohrteil (20) einstückig ausgebildet sind.

2. Trachealkanüle (10) gemäß Anspruch 1, **dadurch gekennzeichnet, dass** der erste Kegelteil (60) und der zweite Kegelteil (70) über einen Übergangsbereich (79) aneinander angrenzen.

3. Trachealkanüle (10) gemäß Anspruch 2, **dadurch gekennzeichnet, dass** die Trageflügel (42, 44) im Wesentlichen im Übergangsbereich (79) angeordnet sind.

4. Trachealkanüle (10) gemäß einem oder mehreren der vorhergehenden Ansprüche, **dadurch gekennzeichnet, dass** die Trageflügel (42, 44) gekrümmt sind.

5. Trachealkanüle (10) nach Anspruch 4, **dadurch gekennzeichnet, dass** ein Radius einer Krümmung (92) der Trageflügel (42, 44) im Wesentlichen in einer Transversalebene (93) anordenbar ist.

6. Trachealkanüle (10) gemäß einem oder mehreren der vorhergehenden Ansprüche, **dadurch gekennzeichnet, dass** die Trageflügel (42, 44) ausgehend von einer Anschlussstelle (95) am Konnektorteil (40) sich nach distal (d) und nach lateral (l) erstrecken.

7. Trachealkanüle (10) gemäß Anspruch 6, **dadurch gekennzeichnet, dass** ein von der Anschlussstelle (95) abgewandtes Ende (45) der Trageflügel (42, 44) einen Abstand (90) zu einer Frontalebene (81), auf der eine im Übergangsbereich (79) angeordnete Übergangsstelle (80) angeordnet ist, umfasst.

8. Trachealkanüle (10) gemäß Anspruch 7, **dadurch gekennzeichnet, dass** der Abstand (90) etwa einer Breite (52) mindestens einer in den Tragflügeln (42, 44) angeordneten Bandausnehmung (46, 48) entspricht.

9. Trachealkanüle (10) gemäß einem oder mehreren der vorhergehenden Ansprüche, **dadurch gekennzeichnet, dass** zumindest das Kanülenrohrteil (20) eine Härte von etwa 60 Shore-A bis etwa 80 Shore-A nach DIN ISO 7619-1 (2012-02) umfasst.

## Claims

1. Tracheal cannula (10) for laryngectomized and/or tracheotomized persons comprising a cannula tube part ( 20), a connector part (40) and at least two supporting wings (42, 44) for fastening a strap, wherein the supporting wings (42, 44) are disposed on the connector part (40), wherein the connector part (40) comprises a first conical part (60) and a second conical part (70), wherein the first conical part (60) is disposed on the cannula tube part (20) and widens away from the cannula tube part (20), and wherein the second conical part (70) is disposed on the first conical part (60) and widens towards the first conical part (60), **characterized in that** the connector part (40) and the cannula tube part (20) are formed in one piece.

2. Tracheal cannula (10) according to claim 1, **characterized in that** the first conical part (60) and the second conical part (70) are adjacent to each other via a transition region (79).

3. Tracheal cannula (10) according to claim 2, **characterized in that** the supporting wings (42, 44) are disposed substantially in the transition region (79).

4. Tracheal cannula (10) according to one or more of the preceding claims, **characterized in that** the supporting wings (42, 44) are curved.

5. Tracheal cannula (10) according to claim 4, **characterized in that** a radius of a curve (92) of the supporting wings (42, 44) can be disposed substantially in a transverse plane (93).

6. Tracheal cannula (10) according to one or more of the preceding claims, **characterized in that** the supporting wings (42, 44) extend distally (d) and laterally (l) from a connection point (95) on the connector part (40).

7. Tracheal cannula (10) according to claim 6, **characterized in that** an end (45) of the supporting wings (42, 44) facing away from the connection point (95) comprises a distance (90) to a frontal plane (81) on which a transition point (80) arranged in the transition region (79) is arranged.

8. Tracheal cannula (10) according to claim 7, **characterized in that** the distance (90) corresponds approximately to a width (52) of at least one strap recess (46, 48) arranged in the supporting wings (42, 44).

9. Tracheal cannula (10) according to one or more of the preceding claims, **characterized in that** at least the cannula tube part (20) comprises a hardness of about 60 Shore-A to about 80 Shore-A according to DIN ISO 7619-1 (2012-02).

## Revendications

1. Canule de trachéotomie (10) pour des personnes laryngectomisées et/ou des personnes trachéotomisées, comprenant une pièce tubulaire de canule (20), une pièce de connexion (40) et au moins deux ailes de support (42, 44) pour la fixation d'une bande de support,
dans laquelle la pièce de connexion (40) et la pièce tubulaire de canule (20) sont formées d'une seule pièce,
dans laquelle les ailes de support (42, 44) sont disposées sur la pièce de connexion (40),
dans laquelle la pièce de connexion (40) comprend une première partie conique (60) et une deuxième partie conique (70),
dans laquelle la première partie conique (60) est disposée sur la pièce tubulaire de canule (20) et s' élargit dans une direction s' éloignant de la pièce tubulaire de canule (20), et
dans laquelle la deuxième partie conique (70) est disposée sur la première partie conique (60) et s' élargit dans la direction vers la première partie conique (60).

2. Canule de trachéotomie (10) selon la revendication 1, **caractérisée en ce que** la première partie conique (60) et la deuxième partie conique (70) sont adjacentes l' une à l' autre par une zone de transition (79).

3. Canule de trachéotomie (10) selon la revendication 2, **caractérisée en ce que** les ailes de support (42, 44) sont disposées essentiellement dans la zone de transition (79).

4. Canule de trachéotomie (10) selon l' une ou plusieurs des revendications précédentes, **caractérisée en ce que** les ailes de support (42, 44) sont incurvées.

5. Canule de trachéotomie (10) selon la revendication 4, **caractérisée en ce qu'** un radius d'une courbure (92) des ailes de support (42, 44) peut être disposé essentiellement dans un plan transversal (93).

6. Canule de trachéotomie (10) selon l' une ou plusieurs des revendications précédentes, **caractérisée en ce que** les ailes de support (42, 44) s' étendent distalement (d) et latéralement (l) à partir d'un point de raccordement (95) sur la pièce de connexion (40).

7. Canule de trachéotomie (10) selon la revendication 6, **caractérisée en ce qu'**une extrémité (45) des ailes de support (42, 44) opposée au point de raccordement (95) comprend une distance (90) par rapport à un plan frontal (81) sur lequel est disposé un point de transition (80) disposé dans la zone de transition (79).

8. Canule de trachéotomie (10) selon la revendication 7, **caractérisée en ce que** la distance (90) correspond approximativement à une largeur (52) d'au moins un évidement de bande (46, 48) disposé dans les ailes de support (42, 44).

9. Canule de trachéotomie (10) selon l' une ou plusieurs des revendications précédentes, **caractérisée en ce qu'**au moins la pièce tubulaire de canule (20) comprend une dureté d' environ 60 Shore-A à environ 80 Shore-A selon la norme DIN ISO 7619-1 (2012-02).
